# EUROPEAN PATENT APPLICATION

(11) **EP 4 397 753 A1**
(43) Date of publication of application: **10.07.2024**
(21) Application number: 22864975.2
(22) Date of filing: 29.08.2022
(51) Int. Cl.: C12N 5/079, C12N 5/073, C12N 5/078

(54) **METHOD FOR MASS ISOLATION AND CONCENTRATION OF TISSUE-DERIVED VESICLES**

(30) Priority: 01.09.2021 KR 20210116449
(71) Applicant: CG Health Tech Inc., Wonju-si, Gangwon-do 26460 (KR)
(72) Inventor: LEE, Sam Yeon, Seoul 06375 (KR); YOON, Song Hee, Gwangju-si, Gyeonggi-do 12798 (KR)
(74) Representative: Danubia Patent & Law Office LLC
(86) International application number: PCT/KR2022/012858
(87) International publication number: WO 2023/033473

(57) **Abstract**

The present disclosure relates to a method of separating and concentrating a large number of extracellular vesicles (EVs) from various tissues, and more particularly, to a method of quickly separating and concentrating a large number of extracellular vesicles to be separated from animal and human tissues using multiple washing processes, enzyme reactions, and filters.

According to the method of separating and concentrating a large number of tissue-derived extracellular vesicles (exosomes and ectosomes) of the present disclosure, compared to conventional exosome isolation kits or ultra-high-speed centrifugation methods, extracellular vesicles from tissues may be rapidly separated and concentrated in large quantities.

## Description

### BACKGROUND OF THE DISCLOSURE

### Field of the Disclosure

The present disclosure relates to a method of separating and concentrating a large number of extracellular vesicles (EVs) from various tissues. More particularly, the present disclosure relates to a method of quickly separating and concentrating a large number of extracellular vesicles to be separated from animal and human tissues using multiple washing processes, enzyme reactions, and filters.

### Description of the Related Art

Currently, a new paradigm of adult stem cell therapy to treat incurable diseases is being applied clinically in a variety of ways.

However, there is a problem of zoonosis of xenogenic serum that may occur during the process of extracting stem cells from a patient or donor and culturing the selected stem cells in vitro. Additionally, when stem cells are transplanted into the body, tumors may form due to the high proliferative capacity and relatively large cell size of the stem cells, and problems such as vascular occlusion causing infarcts may occur.

Methods to avoid various risk factors or problems that may arise from treatment using stem cells directly are being actively researched. For example, research results have reported that extracellular vesicles (EVs) derived from stem cells can replace stem cells.

Recently, research has reported that secretomes contain various bioactive factors that control cell behavior. In particular, the secretomes include 'exosomes' or 'extracellular vesicles' that have a signaling function between cells, and research on the components and functions thereof is actively in progress.

Cells release various membrane-type vesicles into the extracellular environment. Typically, these released vesicles are called extracellular vesicles (EVs). Extracellular vesicles are also called membrane-derived vesicles, ectosomes, shedding vesicles, microparticles, and exosomes. In some cases, extracellular vesicles are used separately from exosomes.

Exosomes are vesicles that are made of a double phospholipid membrane similar to the structure of a cell membrane and have a size of tens to hundreds of nanometers, and exosomes contain proteins called exosome cargos and nucleic acids (mRNA and miRNA). Exosome cargos include a wide range of signaling factors, and these signaling factors are known to be cell type specific and differently regulated depending on the secretory cell environment.

Extracellular vesicles are intercellular signaling mediators secreted by cells, and various cell signals transmitted through extracellular vesicles are known to regulate cell behavior, including activation, growth, migration, differentiation, dedifferentiation, apoptosis, and necrosis of target cells.

In addition, the composition of extracellular vesicles varies depending on the nature and state of the cell from which the extracellular vesicles are derived. For example, extracellular vesicles derived from proliferating stem cells are known to regulate cell behavior such as cell migration, proliferation, and differentiation, and reflect the characteristics of stem cells related to tissue regeneration.

Conventional techniques for separating exosomes and/or extracellular vesicles include ultracentrifugation, density gradient centrifugation, ultrafiltration, size exclusion chromatography, ion exchange chromatography, immunoaffinity capture, microfluidics-based isolation, exosome precipitation, a total exosome isolation kit, and polymer-based precipitation.

An example of a method commonly used in the past is a centrifugal separator, which collects vesicles from pellets formed using centrifugal force.

However, the sedimentation velocity of particles in the micron size range is very low and consequently centrifugation of these particles takes minutes to hours. In addition to the above problems, even after pellet formation, a lot of manpower and time are required in the process of releasing the pellets, and sediment may be generated due to agglomeration of vesicles.

In addition, when using the centrifugation method, only about 5 % to 25 % of extracellular vesicles contained in a sample may be separated, and most of the remaining extracellular vesicles (75 % to 95 %) are lost. In addition, equipment required for ultra-high-speed centrifugation is expensive, and there are limitations in that the amount that can be separated at one time is not large.

To solve the above problems, the present inventors sought to develop a method of separating and concentrating a large number of extracellular vesicles with high efficiency in a short period of time.

### SUMMARY OF THE DISCLOSURE

Therefore, the present disclosure has been made in view of the above problems, and it is an object of the present disclosure to provide a method of separating and concentrating a large number of extracellular vesicles from tissues, specifically, a method of quickly separating and concentrating a large number of extracellular vesicles to be separated from tissues using multiple washing processes, enzyme reactions, and molecular weight cut-off (MWCO) membrane filters.

It is another object of the present disclosure to provide a degradative enzyme compound for obtaining a large number of extracellular vesicles.

In accordance with one aspect of the present disclosure, provided is a method of separating and concentrating extracellular vesicles from tissues, the method including (a) performing first washing of tissues to be separated using a washing solution; (b) removing tissue membranes visible to a naked eye using sterilized tweezers after the first washing; (c) performing grinding at 10 to 100,000 rpm for 1 to 60 minutes using tissue-specific grinding equipment; (d) performing second washing using ethanol, acetone, ether, chloroform, isopropanol, or dichloroethane to remove impurities of residual blood, residual membranes, microvessels, lipids, and fibers; (e) performing freeze-drying in a freezing environment for 6 to 240 hours after the second washing; (f) performing treatment with a degradative enzyme compound after the freeze-drying and performing a reaction at 37 °C and 100 to 4,000 rpm for 1 to 60 minutes; (g) removing undigested tissues and collecting only supernatant by performing centrifugation at 100 to 100,000 rpm for 3 to 60 minutes after step (f); (h) filtering the supernatant by repeatedly passing the supernatant through a 0.22 µm filter system 1 to 15 times; and (i) separating and concentrating extracellular vesicles through tangential flow filtration (TFF) using a molecular weight cut-off (MWCO) membrane.

In the present disclosure, the term "extracellular vesicles (EVs)" are used to encompass membrane vesicles, ectosomes, shedding vesicles, microparticles, nanoparticles, or equivalents thereof. Depending on the separation environment, conditions, and methods, extracellular vesicles may have the same meaning as exosomes, or extracellular vesicles may also include nanovesicles that are the same or similar in size to exosomes but do not have the composition of exosomes. In addition, in the present disclosure, the term exosomes, used in relation to production promotion or productivity, may be used to encompass the extracellular vesicles.

In the present disclosure, the term "exosomes" refers to vesicles composed of a double phospholipid membrane identical to the structure of a cell membrane and having a size of tens to hundreds of nanometers (preferably approximately 30 to 500 nm) (However, the particle size of the exosomes may vary depending on the type of cells to be separated, separation methods, and measurement methods) (Vasiliy S. Chernyshev et al., "Size and shape characterization of hydrated and desiccated exosomes", Anal Bioanal Chem, (2015) DOI 10.1007/s00216-015-8535-3). Exosomes contain proteins called exosome cargos and nucleic acids (mRNA, miRNA, etc.).

Exosome cargos include a wide range of signaling factors, and these signaling factors are known to be specific to cell type and regulated differently depending on the environment of the secretory cell. Exosomes are intercellular signaling mediators secreted by cells, and various cell signals transmitted through exosomes are known to regulate cell behavior, including activation, growth, migration, differentiation, dedifferentiation, apoptosis, and necrosis of target cells.

In addition, in the present disclosure, the term "exosomes" include all vesicles (e.g., exosome-like vesicles) that have a nano-sized vesicle structure secreted from cells and released into the extracellular space and have a composition similar to that of exosomes.

In addition, the extracellular vesicles of the present disclosure are isolated and concentrated from tissues. The tissues include one or more selected from the group consisting of tissues such as brain, fat, placenta, umbilical cord, blood, liver, lung, heart, cord blood, kidney, urine, skin (epithelial cells), and cecum derived from animals or humans and lymph nodes. In the present disclosure, the type of animal-derived cells from which exosomes and/or extracellular vesicles are derived is not limited, but may be stem cells or immune cells as an example that does not limit the present disclosure. The stem cells may be embryonic stem cells, induced pluripotent stem cells (iPSCs), adult stem cells, mesenchymal stem cells derived from embryonic stem cells, or mesenchymal stem cells derived from induced pluripotent stem cells. The immune cells may be T cells, B cells, NK cells, cytotoxic T cells, dendritic cells, or macrophages.

The most commonly used method for separation and concentration of extracellular vesicles is centrifugation and using kits. This method is a method of concentrating extracellular vesicles using a polymer called polyethyleneglycol (PEG). A precipitation phenomenon is used in which PEG reduces the solubility of extracellular vesicles, causing the extracellular vesicles to elute and settle. As the reaction time between PEG and extracellular vesicles increases, the number of extracellular vesicles precipitated increases. A reaction time of 4 to 24 hours is usually required. Exo-Quick is known as a representative product that uses the elution method by PEG.

Compared to Exo-Quick, in the case of the method of separating extracellular vesicles according to the present disclosure, the separation capacity increases by 10 to 1,000,000 times, and the separation time decreases to about 1/40 or less. Accordingly, the method of the present disclosure is efficient in terms of productivity (Table 1).

In addition to the sedimentation method, a frequently used method of separating extracellular vesicles is the method using filters. The filter method is a method of filtering out extracellular vesicles using a filter with pores smaller than the size of the extracellular vesicles. Since extracellular vesicles are filtered out and other proteins are excreted, the separation rate may be adjusted depending on the fluid velocity. In addition, since no reaction time is required, the separation time is short. However, since the rate of extracellular vesicles that bind to or escape from the filter is high, the final recovery rate is not high.

The separation and concentration method according to the present disclosure is characterized by overcoming the limitations of the prior art by pretreatment with a degradative enzyme compound and using a membrane manufactured according to the present disclosure.

### BRIEF DESCRIPTION OF THE DRAWINGS

The above and other objects, features and other advantages of the present disclosure will be more clearly understood from the following detailed description taken in conjunction with the accompanying drawings, in which:
FIG. 1 shows a flowchart for explaining a method of separating extracellular vesicles from tissues according to the present disclosure;
FIG. 2 shows fluorescence images taken after isolating extracellular vesicles from any three tissues under different conditions;
FIG. 3 shows transmission electron microscopy (TEM) results obtained after isolating extracellular vesicles from any three tissues under different conditions;
FIG. 4 shows western blot results obtained after isolating extracellular vesicles from any three tissues under different conditions;
FIG. 5 shows the results of confirming the particle size and concentration of extracellular vesicles separated and concentrated by the method of the present disclosure by nanoparticle tracking analysis (NTA); and
FIG. 6 shows a heatmap of RNA-Seq profiling results (row: grouping of target factors among RNA sequencing results, column: extracellular vesicles extracted from each sample).

### DETAILED DESCRIPTION OF THE DISCLOSURE

Hereinafter, the present disclosure is described in detail by examples. However, the following examples only illustrate the present disclosure, and the present disclosure is not limited by the following examples.

### Example 1. Separating and concentrating extracellular vesicles from tissues

In the present disclosure, tissues for isolation and enrichment of extracellular vesicles include one or more selected from the group consisting of brain, fat, placenta, umbilical cord, blood, liver, lung, heart, cord blood, kidney, urine, skin (epithelial cells), cecum and lymph nodes derived from animals or humans. In this example, three types of tissues (brain, placenta, and blood) were used.

First, tissues to be separated were subjected to first washing using a washing solution to remove blood and other impurities in tissues. At this time, the washing solution may include one or more selected from the group consisting of phosphate-buffered saline (PBS), Hanks' balanced salt solution (HBSS), Earle's balanced salt solution (EBSS), normal saline, 5 % dextrose water, and Hartman solution.

After the first washing, tissue membranes visible to the naked eye were removed using sterilized tweezers, and grinding was performed at 10 to 100,000 rpm for 1 to 60 minutes using tissue-specific grinding equipment. At this time, as the tissue-specific grinding equipment, equipment that may be sterilized for each part and has a pressing frame function using gravity was used, allowing different rpm to be applied depending on the area or type of each tissue.

In addition, the tissue membrane observed with the naked eye may include one or more selected from the inner membrane, outer membrane, tendon, and double membrane of a tissue.

After the grinding, second washing was performed using ethanol, acetone, ether, chloroform, isopropanol, or dichloroethane to remove impurities such as residual blood, residual membranes, microvessels, lipids, and fibers.

After the second washing, freeze-drying was performed for 6 to 120 hours in a freezing environment.

After the freeze-drying, treatment with a degradative enzyme compound was performed, and a reaction was performed at 37 °C and 100 to 4,000 rpm for 1 to 60 minutes. At this time, the degradative enzyme compound includes one or more enzymes selected from the group consisting of collagenase I, collagenase II, collagenase III, collagenase IV, collagenase V, dispase I, dispase II, DNase, trypsin, papain, endopeptidase, elastase, protease, and pectinase. Specifically, the degradative enzyme compound may be an enzyme including collagenase I in a ratio of 1 % to 100 %, collagenase II in a ratio of 1 % to 100 %, collagenase III in a ratio of 1 % to 100 %, collagenase IV in a ratio of 1 % to 100 %, collagenase V in a ratio of 1 % to 100 %, dispase I in a ratio of 1 % to 100 %, dispase II in a ratio of 1 % to 100 %, DNase in a ratio of 1 % to 100 %, trypsin in a ratio of 1 % to 100 %, papain in a ratio of 1 % to 100 %, endopeptidase in a ratio of 1 % to 100 %, elastase in a ratio of 1 % to 100 %, proteases in a ratio of 1 % to 100 %, and pectinase in a ratio of 1 % to 100 %. Each enzyme may be prepared at a concentration of 1 µg/ml to 100 mg/ml or 0.1 U/ml to 100 U/ml and mixed.

More specifically, in the degradative enzyme compound, the ratio of collagenase I may be 1 % to 100 %, 5 to 95 %, 10 to 80 %, preferably 10 to 80 %. The ratio of collagenase II may be 1 % to 100 %, 10 to 80 %, 40 to 65 %, preferably 33 to 65 %. The ratio of collagenase III may be 1 % to 100 %. The ratio of collagenase IV may be 1 % to 100 %, 3 to 95 %, 5 to 90 %, 10 to 80 %, preferably 10 to 80 %. The ratio of dispase I may be 1 % to 100 %, 10 to 80 %, 30 to 75 %, 35 to 65 %, preferably 35 to 65 %. The ratio of dispase II may be 1 % to 100 %. The ratio of DNase may be 1 % to 100 %, 3 to 95 %, 5 to 90 %, 10 to 85 %, preferably 10 to 80 %. The ratio of trypsin may be 1 % to 100 %, 3 to 95 %, 5 to 90 %, 10 to 85 %, preferably 10 to 80 %. The ratio of papain may be 1 % to 100 %. The ratio of endopeptidase may be 1 % to 100 %. The ratio of elastase may be 1 % to 100 %. The ratio of proteases may be 1 % to 100 %, 5 to 90 %, 5 to 85 %, 10 to 80 %, preferably 10 to 80 %. The ratio of pectinase may be 1 % to 100 %. The present disclosure is not limited thereto.

After treatment with the degradative enzyme compound, undigested tissues were removed and only supernatant was collected by performing centrifugation at 100 to 100,000 rpm for 3 to 60 minutes.

Then, the supernatant was filtered by repeatedly passing the supernatant through a 0.22 µm filter system 1 to 15 times, and then extracellular vesicles were separated and concentrated through tangential flow filtration (TFF) using a molecular weight cut-off (MWCO) membrane of 3 to 5,000 kda.

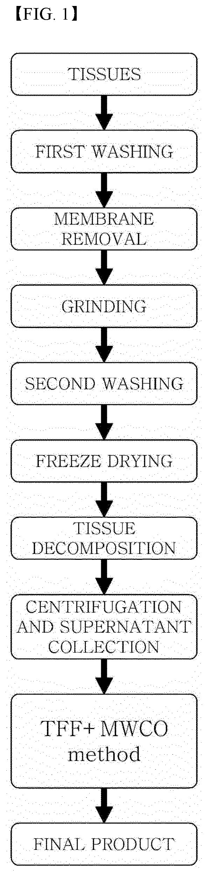

This entire process is shown in FIG. 1.

### Experimental Example 1. Comparison of efficiency depending on separation conditions of extracellular vesicles

The efficiency of the process conditions in Example 1 was compared with the efficiency of the conventional method of separating extracellular vesicles.

Specifically, for the conventional ultracentrifugation and Exo-Quick conditions and the separation method in Example 1 of the present disclosure, protein quantification was performed using a BCA (bicinchoninic acid) reagent, and the particle number was measured by Nanoparticle Tracking Analysis (NTA), and the results are shown in Table 1 below.

**[Table 1]**

| Conditions | Separation time | BCA results (Protein) | NTA results (Particle number) |
|---|---|---|---|
| Ultracentrifugation | 5 hours | 50 to 100 µg | 1 × 10⁹ to 1 × 10¹⁰ |
| Exo-Quick | 13 hours | 75 to 200 µg | 1 × 10¹⁰ to 6 × 10¹⁰ |
| Example 1 | 20 minutes | 75 to 200 µg | 1 × 10¹⁰ to 1 × 10¹¹ |

At the same capacity, compared to ultracentrifugation and Exo-Quick, when using the process of Example 1 of the present disclosure, the separation time was reduced by about 1/15 to 1/40. In addition, when protein quantification was performed using the BCA method, ultracentrifugation showed the lowest value, and Exo-Quick and the method of the present disclosure showed similar results. Also, when the particle number was measured, ultracentrifugation showed the lowest value, and Exo-Quick and the method of the present disclosure showed similar results.

### Experimental Example 2. Identification of separated extracellular vesicles

It was confirmed whether extracellular vesicles could actually be obtained from tissues.

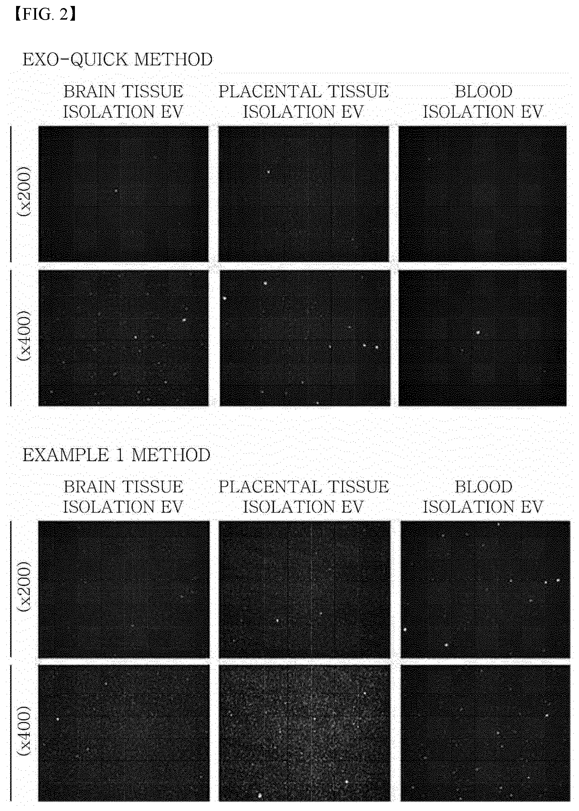

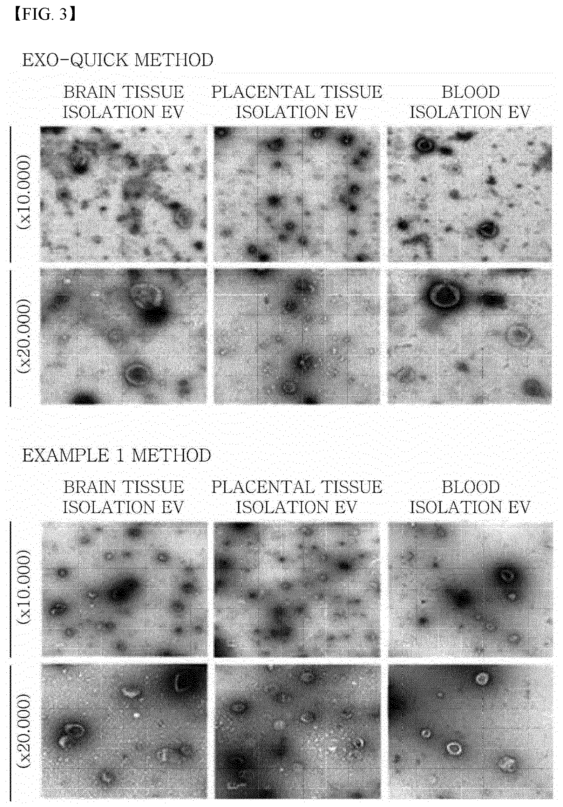

Specifically, extracellular vesicles were separated and concentrated from brain, placenta, and blood using Exo-Quick and the method of Example 1. Afterwards, to confirm that extracellular vesicles were indeed obtained, fluorescence imaging analysis (FIG. 2) and transmission electron microscopy analysis (FIG. 3) were performed. The images obtained by the conventional method (Exo-Quick) and the images obtained by the method of Example 1 were compared.

Specifically, using immunocytochemistry (Images obtained by staining with CD63, an exosome marker, and magnifying 200 and 400 times with a fluorescence microscope) and a transmission electron microscope (Images obtained by processing with specific reagents for transmission electron microscopy and magnifying 10,000 times and 20,000 times), extracellular vesicles were observed for both methods, Exo-Quick and Example 1. It was confirmed that a greater number of extracellular vesicles were observed in the method of Example 1. It was confirmed that extracellular vesicles could be separated and concentrated in large quantities according to the separation and concentration method of the present disclosure under the conditions.

### Experimental Example 3. Analysis and productivity evaluation of separated extracellular vesicles by nanoparticle tracking analysis

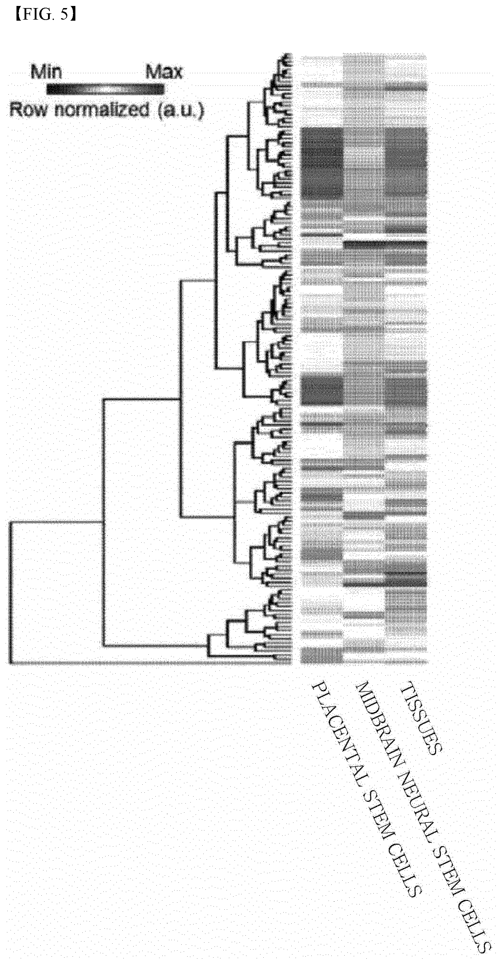

The particle size and concentration of extracellular vesicles obtained using the method of the present disclosure were measured by nanoparticle tracking analysis, and the results are shown in FIG. 5.

As shown in FIG. 5, the sizes of the extracellular vesicles obtained using the method of the present disclosure were very uniform, and the extracellular vesicles had an average size of 134.3 nm. The total concentration was 1.1 × 10 ¹³ particles/ml, and the yield per sample was 6 × 10 ¹¹ particles/ml.

That is, according to the method of the present disclosure, extracellular vesicles of uniform size distributed over a certain range may be obtained. In this case, damage and deformation of extracellular vesicles may be minimized, and a large number of extracellular vesicles may be isolated from the same amount of tissues.

In addition, the total concentration and calculated values per sample show significantly improved results compared to Exo-Quick (exosome isolation kit) and ultracentrifugation, which are values obtained by the conventional methods.

Therefore, the present disclosure may significantly improve productivity by providing a method of effectively separating and concentrating extracellular vesicles released from tissues. Therefore, the method of the present disclosure may be useful commercially and clinically. In addition, the manufacturing method in the present disclosure may economically and efficiently produce extracellular vesicles with high yield. In particular, compared to the conventional methods, the method of the present disclosure has the advantage of being able to produce extracellular vesicles in large quantities.

### Experimental Example 4. Experiment comparing components of extracellular vesicles isolated from tissue and cell media

The present disclosure is characterized by obtaining extracellular vesicles at high concentration and high purity from tissues that can generally be obtained in large quantities, and this remarkable improvement in productivity is as described above.

In addition, the present inventors sought to confirm whether extracellular vesicles obtained directly from tissues using the method exhibit the same composition as extracellular vesicles obtained after culturing stem cells.

Specifically, the composition of extracellular vesicles obtained from human placenta stem cells and midbrain neural stem cells using the Exo-Quick method was compared with the composition of extracellular vesicles obtained in Example 1 (FIG. 6, RNA-Seq profiling result heatmap).

In this experimental example, the expression levels of inflammation-related factors, cell growth factors, and antioxidant factors, which are characteristics of stem cells, were analyzed using RNA sequencing (RNA-Seq), and the results were displayed through a heatmap. The row shows the groups of targeted factors among the RNA sequencing results, and the column shows extracellular vesicles extracted from each sample. Relatively high expression is shown in red and relatively low expression is shown in blue.

As a result, as shown in FIG. 6, expression of similar inflammation-related factors was confirmed in all three extracellular vesicles. The composition of extracellular vesicles extracted from tissues using the method developed in this patent was about 95 % or more identical to the composition of extracellular vesicles obtained from cultured stem cells using the existing commercially available Exo-Quick.

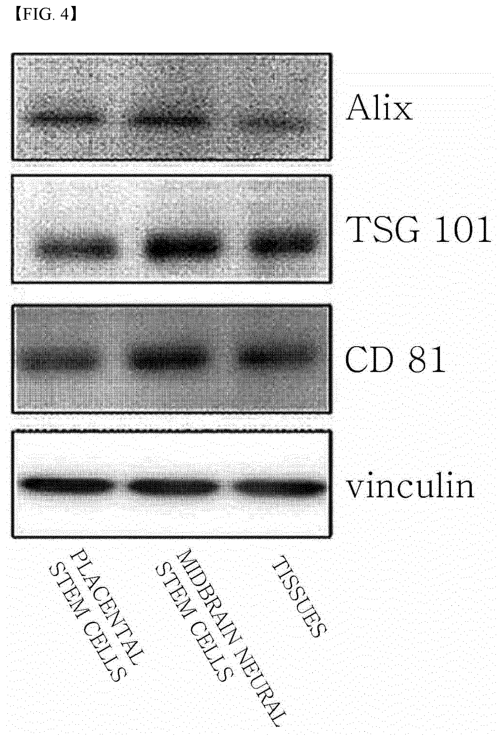

In addition, western blot was performed on extracellular vesicles separated from placenta stem cells, midbrain neural stem cells, and tissues under the conditions of Example 1 of the present disclosure to confirm the expression of Alix (or PDCD6IP), TSG 101, CD81, and vinculin, which are known as protein markers of exosomes. As a result, as shown in FIG. 4, it was confirmed that all protein markers were expressed.

According to the method of separating and concentrating a large number of extracellular vesicles of the present disclosure, exosomes and/or extracellular vesicles that can be commercially and/or clinically useful can be produced economically and efficiently with high yield. In particular, compared to conventional exosome isolation kits or ultra-high-speed centrifugation methods, a large number of exosomes isolated from tissues can be quickly separated and concentrated.

The effects of the present disclosure are not limited to the above-described effects, and should be understood to include all effects that can be inferred from the configuration of the invention described in the detailed description or claims of the present disclosure.

The aforementioned description of the present disclosure is provided by way of example and those skilled in the art will understand that the present disclosure can be easily changed or modified into other specified forms without change or modification of the technical spirit or essential characteristics of the present disclosure. Therefore, it should be understood that the aforementioned examples are only provided by way of example and not provided to limit the present disclosure. For example, each of constituents described as a single form may be separately implemented and, similarly, constituents described as being separated may be implemented in a combined form.

It should be understood that the scope of the present disclosure is defined by the following claims and the invention is to cover all modifications, equivalents, and alternatives falling within the spirit and scope of the invention as defined by the claims.

## Claims

1. A method of separating and concentrating extracellular vesicles from tissues, comprising:
(a) performing first washing of tissues to be separated using a washing solution;
(b) removing tissue membranes visible to a naked eye using sterilized tweezers after the first washing;
(c) performing grinding at 10 to 100,000 rpm for 1 to 60 minutes using tissue-specific grinding equipment;
(d) performing second washing using ethanol, acetone, ether, chloroform, isopropanol, or dichloroethane to remove impurities of residual blood, residual membranes, microvessels, lipids, and fibers;
(e) performing freeze-drying in a freezing environment for 6 to 240 hours after the second washing;
(f) performing treatment with a degradative enzyme compound after the freeze-drying and performing a reaction at 37 °C and 100 to 4,000 rpm for 1 to 60 minutes;
(g) removing undigested tissues and collecting only supernatant by performing centrifugation at 100 to 100,000 rpm for 3 to 60 minutes after step (f);
(h) filtering the supernatant by repeatedly passing the supernatant through a 0.22 µm filter system 1 to 15 times; and
(i) separating and concentrating extracellular vesicles through tangential flow filtration (TFF) using a molecular weight cut-off (MWCO) membrane.

2. The method according to claim 1, wherein the washing solution comprises one or more selected from the group consisting of phosphate-buffered saline (PBS), Hanks' balanced salt solution (HBSS), Earle's balanced salt solution (EBSS), normal saline, 5 % dextrose water, and Hartman solution.

3. The method according to claim 1, wherein the tissue membrane observed with a naked eye comprises one or more selected from an inner membrane, outer membrane, tendon, and double membrane of a tissue.

4. The method according to claim 1, wherein the degradative enzyme compound consists of one or more enzymes selected from the group consisting of collagenase I, collagenase II, collagenase III, collagenase IV, collagenase V, dispase I, dispase II, DNase, trypsin, papain, endopeptidase, elastase, protease, and pectinase.

5. The method according to claim 1, wherein the molecular weight cut-off membrane is a cut-off membrane of 3 kDa to 5,000 kda.

6. Extracellular vesicles obtained by the method according to any one of claims 1 to 5.
